# EUROPEAN PATENT APPLICATION

(11) **EP 3 459 534 A1**
(43) Date of publication of application: **27.03.2019**
(21) Application number: 17306239.9
(22) Date of filing: 22.09.2017
(51) Int. Cl.: A61K 31/00

(54) **METHOD FOR TREATING OR PREVENTING RADIOTHERAPY INDUCED BREAST FIBROSIS**

(71) Applicant: Institut Regional du Cancer de Montpellier, 34090 Montpellier (FR)
(72) Inventor: AZRIA, David, 30250 FONTANES (FR)
(74) Representative: Flesselles, Bruno F.G.

(57) **Abstract**

The invention relates to the field of therapy and prevention of radiotherapy-induced fibrosis, in particular after breast cancer radiotherapy, by administering a Rho kinase inhibitor to patients, in particular with a predetermined high risk of such fibrosis.

## Description

The invention relates to the field of therapy and prevention of radiotherapy-induced fibrosis, in particular after breast cancer radiotherapy.

The primary target of radiation therapy is the eradication of microscopic residual disease adjacent to the original site of the tumor as well as the elimination of any evidence of multicentric disease. External beam radiation is the standard treatment of breast cancer patients who underwent breast conserving surgery. Adjacent structures (such as heart, lung, etc...) in addition to tumor bed and whole breast are exposed to ionizing radiation (1).

Radiation-induced complications can be classified into 2 groups based on the time sequence of occurrence (Table 1 of Yi et al, Korean J Radiol. 2009 Sep-Oct; 10(5): 496-507).
(a) early toxicities that occur during radiotherapy course and during the first 3 months following completion of radiation therapy (mainly erythema).
(b) late complications that occur at least 6 months after radiotherapy completion (fibrosis, bone fracture, lung fibrosis, cardiomyopathy, radiation-induced malignancies, etc...).

Breast fibrosis refers to a thickening and stiffening of tissues. Inflammation precedes fibrosis occurrence. Breast fibrosis can occur up to 10 years after radiation therapy is completed.

There is no specific treatment for either inflammation or breast fibrosis once established, as fibrosis is a permanent modification in the breast tissue. Thus, antifibrotic treatment (so-called mitigator) is essentially aimed at preventing onset of further fibrosis.

Vitamin E and pentoxifylline have been tested in clinical trials (Delanian et al J Clin Oncol 23:: 2005, 8570-8579).

Some reports have appeared describing improvement of radiation-induced fibrosis, soft-tissue necrosis, and other injury with the use of pentoxifylline and interferon-gamma-1b (Actimmune) and that interferon-gamma inhibits collagen production in dermal fibroblasts 'found to be increased in cutaneous radiation fibrosis) and objective improvement of radiation fibrosis and healing of radiation ulcers and fistulae with the use of low dose interferon-gamma in patients (Gottlober et al Int J Radiat Oncol Biol Phys 50:159-166, 2001; Peter et al Int J Radiat Oncol Biol Phys 45:147-152, 1999).

Pentoxifylline is a methylxanthine derivative that lowers blood viscosity, improves erythrocyte flexibility, and increases oxygen tension levels. Futran et al (Laryngoscope 107:391-395, 1997) reported soft-tissue necrosis resolved completely in 75% of patients with late radiation injury, treated with oral pentoxifylline, improvement of fibrosis in 67%, and mucosal pain resolved in all patients, whereas soft-tissue necrosis progressed to osteoradionecrosis in non-treated patients. Delanian et al (J Clin Oncol 17:3283-3290, 1999) used a combination of penytoxifylline at 800 mg/d and vitamin E (tocopherol) at 1,000 IU/d in patients with radiation-induced fibrosis following treatment for head and neck and breast cancers. Treatment was continued for at least 6 months, and continuous clinical regression of all late effects was noted in all areas studied.

Rho-associated protein kinase (ROCK) is a kinase belonging to the AGC (PKA/PKG/PKC) family of serine-threonine kinases. It is involved mainly in regulating the shape and movement of cells by acting on the cytoskeleton.

ROCK plays a role in a wide range of different cellular phenomena, as ROCK is a downstream effector protein of the small GTPase Rho, which is one of the major regulators of the cytoskeleton.

Recent research has shown that ROCK signaling plays an important role in many diseases including diabetes, intracerebral hemorrhage, neurodegenerative diseases such as Parkinson's disease and amyotrophic lateral sclerosis, pulmonary hypertension and cancer. It has been shown to be involved in causing tissue thickening and stiffening around tumors in a mouse model of skin cancer, principally by increasing the amount of collagen in the tissue around the tumor. Its involvement in lung fibrosis has also been proposed (Knipe et al Pharmacological Reviews January 2015, 67 (1) 103-117).

ROCK inhibitors are thus being developed for treating these diseases and these drugs have potential to prevent cancer from spreading by blocking cell migration, stopping cancer cells from spreading into neighboring tissue.

HMG-CoA reductase inhibitors (statins) are cholesterol-lowering drugs widely known and used in the art. It has also been shown that the molecules are able to interact with the Rho protein and inhibit the Rho-signaling pathway and in particular the Rho kinase (Brandes, Circ Res. 2005;111:927-929; Martin et al, J. Clin. Invest. 107:1423-1432 (2001); Nohria et al, Atherosclerosis. 2009 August ; 205(2): 517-521; Rawlings et al, Am J Cardiol. 2009 February 15; 103(4): 437-441; Shiga et al, Circ Res. 2005;96:1014-1021)

### SUMMARY OF INVENTION

The present invention provides novel uses of ROCK inhibitors for treating and/or preventing fibrosis in patient having received a radiation therapy for treating breast cancer. Such ROCK inhibitors can also be used for in patient during the period in which they are receiving such radiation therapy for breast cancer and can also be used and or started in patients before they start to receive the radiation therapy for breast cancer treatment.

Inhibition of the ROCK (Rho kinase) can be a direct inhibition (by direct action on the enzyme) or an indirect inhibition (in particular by action on the Rho protein, thus leading to loss of activity of the cascade inducing the Rho kinase).

### Rho kinase inhibitor

The Rho kinase inhibitor can be any molecule known in the art one can cite Liao et al (J Cardiovasc Pharmacol. 2007 July; 50(1): 17-24) and Feng et al (J Med Chem. 2016 Mar 24;59(6):2269-300) which list Rho kinase inhibitors.

One can cite, in particular (see Liao et al J Cardiovasc Pharmacol. 2007 Jul; 50(1): 17-24 for further information on these molecules) Fasudil (Intravenous and oral formulation), Y27632, Y39983 (Ophthalmic liquid formulation), Wf-536, SLx-2119, Azabenzimidazole-aminofurazans (ROCK inhibitor, GSK), DE-104, Olefins, Isoquinolines, Indazoles, pyridinealkene derivatives, H-1152P, ROKα inhibitor (ROCK inhibitor, BioFocus), XD-4000, HMN-1152, 4-(1-aminoalkyl)-N-(4-pyridyl)cyclohexane-carboxamides (Oral formulation), Rhostatin (Drug implant, recombinant protein), BA-210, BA-207, BA-215, BA-285, BA-1037, Ki-23095, VAS-012, ROCK Inhibitor (quinazoline). One can also cite Ripasudil (used in Japan for the treatment of glaucoma and ocular hypertension), RKI-1447 (Patel et al Cancer Res. 2012 Oct 1;72(19):5025-34), Y-27632 (Peh et al, Sci Rep. 2015; 5: 9167), Y-30141 (4-(1-aminoethyl)-N-(1H-pyrrolo(2,3-b)pyridin-4-yl)cyclohexanecarboxamide dihydrochloride) and GSK429286A (C₂₁H₁₆F₄N₄O₂, N-(6-fluoro-1H-indazol-5-yl)-2-methyl-6-oxo-4-(4-(trifluoromethyl)phenyl)-1,4,5,6-tetrahydropyridine-3-carboxamide).

These molecules have essentially been developed for cerebral vasospasm, acute stroke, angina, pulmonary hypertension, Reperfusion injury, hypertension, stroke, asthma, cancer, glaucoma, atherosclerosis, fibrosis, solid tumors, hypertension, anti-inflammatory, asthma, CV disease, erectile dysfunction, glaucoma, HIV infection, osteoporosis, hearing loss, tinnitus, hypertension, cerebrovascular disease, neurodegenerative diseases, brain tumors, macular degeneration, spinal cord injury, cardiovascular disease, macular degeneration, renal disease.

The above-mentioned molecules are direct Rho kinase inhibitors (i.e. they directly inhibit the activity of Rho kinase through direct binding to this enzyme).

However, there are also indirect Rho kinase inhibitors, i.e. molecules that lead to inhibition of the activity of the Rho kinase, potentially through interaction with another molecule than the Rho kinase protein, such as with the Rho protein.

As seen above, HMG-CoA reductase inhibitors (statins) eventually lead to inhibition of the Rho kinase and are thus considered as Rho kinase inhibitors.

### HMG-CoA reductase inhibitor

The HMG-CoA reductase inhibitor (statin) can be chosen in the group consisting of atorvastatin, cerivastatin, fluvastatin, lovastatin, mevastatin, pitavastatin, pravastatin, rosuvastatin and simvastatin.

It is preferred when the statin is pravastatin (CAS 81093-37-0). It is preferably used at regimen and dosages used for the classical use for lowering cholesterol.

Consequently, the preferred daily dose of pravastatin is comprised between 10 mg and 50 mg. Most preferably, it is 20 mg or 40 mg. These dosages are in the therapeutic range that makes pravastatin usable for treatment or prevention of fibrosis induced by radiation therapy for patient thus treated for breast cancer.

A preferred therapeutically effective dosage is therefore about 40 mg / day. It is to be noted, however, that the physician may adjust the dosage depending on the patient to be treated (in particular the weight on the patient).

### Patients

The Rho kinase inhibitor, in particular the HMG-CoA reducatse inhibitor, and especially pravastatin can be administered to patients
(a) that have already been treated by radiation therapy for breast cancer
(b) that are being treated by radiation therapy for breast cancer
(c) that will be treated by radiation therapy for breast cancer.

The patient may not yet have developed fibrosis when the treatment is started, as it is a purpose of the treatment to prevent onset of fibrosis or limit the development of fibrosis (limitation of the development of fibrosis can be assessed by comparing the amount of fibrosis of a cohort of patients having received the Rho kinase inhibitor and a cohort of patients not having received the Rho kinase inhibitor).

It is also possible that fibrosis may have already appeared for the patient when treatment with the Rho kinase inhibitor is started. In this case, the treatment would essentially aim at stopping further evolution of fibrosis, although it could also revert fibrosis, in particular by reduction of the fibrosis thickness.

It is however preferred when the treatment with the Rho kinase inhibitor is started before onset of fibrosis. Consequently, it is possible for the patient to follow the treatment before the start of the radiation therapy, during said therapy and for some time after said therapy.

In a specific embodiment, treatment with the Rho kinase inhibitor is started at the same time than the radiotherapy starts. In this embodiment, the patient would have the first take of the Rho kinase inhibitor the day he receives the first radiation dose.

In another embodiment, treatment with the Rho kinase inhibitor is started from one month to one day before the radiotherapy starts.

In any case, it is preferred when the patient receives the treatment during the whole duration of the radiotherapy treatment.

It is also envisaged that treatment with the Rho kinase inhibitor is continued after the end of the radiotherapy, in particular for as long as one year after receipt of the last radiation dose.

When the treatment with the Rho kinase inhibitor is applied during and potentially for some time after radiotherapy, it is postulated that it will change the response of the cells of the patient to the stress due to the radiation therapy, by inhibiting the Rho kinase pathway. Consequently, injuries to the patient's tissue would be reduced and that would thus prevent or limit later apparition of the fibrosis.

It is also preferred to use the treatment in patients that are the most susceptible to develop breast late effect (BLE) due to radiation therapy.

In particular, the patients are chosen in patients for which the RILA (Radiation Induced Lymphocyte Apoptosis) score (see Azria et al, 2015, EBioMedicine 2(12): 1965-1973 and example 1) indicates a susceptibility for breast fibrosis after radiotherapy. In this article, it was shown that radiosensitivity assay based on flow cytometric assessment of RILA, can significantly predict differences in breast fibrosis between individuals and detect hyper-reactive patients to RT. Negative predictive value (representing the number of true negative people in people negative to the test) was found in case of high RILA value and less grade breast fibrosis (Ozsahin et al 2005 Clin Cancer Res 11(20): 7426-7433.). In addition, all severe breast fibrosis (grade >2) were observed in patients with tow values of RILA.

In summary, the RILA test consists in irradiating lymphocytes of the patient and detecting the apoptosis rate. In particular a dose of 8Gy is administered to lymphocytes (preferably a 1Gy/mn) on lymphocytes of a blood sample (in the first 24 hours after harvest) and apoptosis rate is determined after 48 hours of sample incubation after irradiation. The lymphocytes for which apoptosis rate is evaluated are preferably CD8 lymphocytes. Apoptosis rate is evaluated by methods known in the art, and in particular using flow cytometry. Example 1 further provides information on a protocol that can be used to assess the rate of lymphocyte.

Patients with an CD8 lymphocytes apoptosis rate lower than 20 %, preferably lower than 18 %, or even lower than 16 % or 14 % are particularly susceptible to be treated by a Rho kinase inhibitor according to the teachings of herein disclosed.

Although informative and usable in the context for selecting patients most appropriate to receive the treatment herein disclosed, RILA may not be the best assay in terms of sensitivity and specificity.

In particular, another method to predict the risk of developing Breast Late Effects (BLE) and in particular fibrosis after RT can be used.

This method combines the RILA method and the use of further clinical parameters, namely tobacco smoking habits and adjuvant hormonotherapy of the patient.

The RILA outcome and the two clinical parameters are combined through a multivariate Cox function to obtain an end value to determine the risk of developing BLE.

By "tobacco smoking habits" it is meant that tobacco smoking patient or non-smoking patients as defined hereafter. A non-smoking patient is defined as some-day smoker or never daily smoker (and given value=0 in the multivariate Cox function). Any other tobacco smoking patient (daily smoker, intermittent smoker or non-daily smoker) is given a value=1 in the multivariate Cox function.

A "some-day smoker" is a subject having ever smoked 100 cigarettes during the smoker's lifetime and currently smoking on some days (not every day).

A "never daily smoker" is a subject having never smoked daily for 6 months or more.

An "adjuvant hormonotherapy" is a hormonal treatment that given after surgery, chemotherapy, and/or radiation therapy to lower the chance of recurrence of the cancer. Hormone receptor-positive breast cancer depends on hormones called estrogen and/or progesterone to grow. Adjuvant hormonal therapy allows to lower the levels of these hormones in the body or to block the hormones from getting to any remaining cancer cells. Hormonal therapy for hormone receptor-positive breast cancer are selected from therapies using medications selected from the group consisting of tamoxifen, aromatase inhibitors (Als), such as anastrozote (Arimidex), exemestane (Aromasin), and letrozole (Femara), and ovarian suppression by surgery or by drugs selected from gonadotropin, luteinizing, goseretin (Zotadex) and leuprolide (Lupron).

Multivariate Cox regression is one of the usual statistical model for time-to-event analysis. Apart from a classification algorithm which directly deals with binary or multi-class outcomes, multivariate Cox regression defines a semi-parametric model to directly relate the predictive variables with the real outcome, which is in this case the appearance of a BLE and in particular fibrosis (e.g., in months or years). Multivariate Cox function is the best hazard function in terms of discrimination for time-to event endpoint to combine independent parameters. The independent parameters are RILA result (percentage of lymphocytes undergoing apoptosis), adjuvant hormonotherapy and tobacco smoking habits. The multivariate Cox function is obtained by combining the relative weight of each parameter, as individually determined in the multivariate Cox regression, with a negative sign when the markers harbor a negative correlation with the observation of fibrosis. In the present invention, the classification of the patients is based on the occurrence of BLE during clinical follow-up of studies.

In particular, the Multivariate Cox function (experiencing the BLE) = baseline hazard *exp(β1*RILA + β2*(Adjuvant Hormonotherapy [0=no; 1=yes] + β3*(Tobacco smoking habits[0=no; 1=yes]), wherein :
- β1 is comprised between -0.077 and -0.010;
- β2 is comprised between 0.283 and 1.980;
- β3 is comprised between -0.063 and 0.965.

This Cox function can be obtained from the results obtained from a cohort of patients (at least 100 patients), through the following steps
i) Assessing the presence of BLE in each patients, wherein the following data are known for each patient
   a. The RILA result
   b. The tobacco smoking habits
   c. The presence of adjuvant hormonotherapy; and
ii) Performing a multivariate Cox regression by combination of said data a., b. and c.,
iii) Analyzing said multivariate Cox regression to assess the independent discriminative value of said data a., b. and c.,
iv) Combining the relative weight of each data a., b. and c., as individually determined in the multivariate Cox regression, with a negative sign when the markers harbor a negative correlation with the observation of BLE.

When risk of developing a BLE after radiotherapy is more than 8% (cut-off value obtained including all independent predictive factors, corresponding to a probability of a 3y-BF-FS higher than 0.92), it is considered a significative risk.

Treatment with the Rho kinase inhibitor is thus preferably administered to patients having such risk.

Also subjects of the invention are compositions containing a Rho kinase inhibitor for the use thereof in the treatment or prevention of fibrosis induced by radiotherapy of breast cancer, and also the use of such inhibitors for the production or manufacture of a drug intended for the treatment or prevention of fibrosis induced by radiotherapy of breast cancer.

The teachings of the invention thus make it possible to implement treatment methods comprising the administration of a Rho kinase inhibitor to patients having undergone a radiotherapy treatment of breast cancer.

The teachings of the invention thus make it possible to implement methods for preventing onset of fibrosis (or reducing the severity of future fibrosis) comprising the administration of a Rho kinase inhibitor to patients during a radiotherapy treatment of breast cancer.

The teachings of the invention thus make it possible to implement methods for preventing onset of fibrosis (or reducing the severity of future fibrosis) comprising the administration of a Rho kinase inhibitor to patients before they start a radiotherapy treatment of breast cancer.

The invention relates to a method for treating fibrosis induced by breast cancer radiotherapy, comprising the step of administering to a patient in need thereof a therapeutic amount of a Rho kinase inhibitor.

The invention relates to a method for preventing or limiting the development of fibrosis induced by breast cancer radiotherapy comprising the step of administering an effective amount of a Rho kinase inhibitor to a patient prior to, during or after breast cancer radiotherapy treatment.

The invention relates to the use of a Rho kinase inhibitor for the preparation of a medicament intended for treatment or prevention of fibrosis induced by breast cancer radiotherapy.

The invention thus also relates to a method for administering a Rho kinase inhibitor to a patient in need thereof (whether having received, receiving or set to receive a radiation treatment for breast cancer). Such Rho kinase inhibitor is administered at a therapeutically active dosage.

The therapeutic effect observed may be:
(a) no start of development of fibrosis
(b) less fibrosis observed than in patients that would not have received the Rho kinase inhibitor
(c) stop of development of fibrosis for patients in which such fibrosis has already started
(d) any degree of regression of already present fibrosis.

The Rho kinase inhibitor can be used alone or in combination with another compound used for treating or preventing fibrosis (or symptoms thereof).

The invention therefore covers a composition containing a Rho kinase inhibitor and also another medicament against fibrosis, for simultaneous, separate or sequential (spread out over time) use in the treatment or prevention of fibrosis after radiation therapy of breast cancer. Such other medicament can be another Rho kinase inhibitor. In particular, it is foreseen when one uses a HMG-CoA reductase inhibitor (in particular pravastatin) and another Rho kinase inhibitor that is not a HMG-CoA reductase inhibitor, but a Rho kinase inhibitor as mentioned above. One can also provide a Rho kinase inhibitor (in particular a HMG-CoA reductase inhibitor and especially pravastatin) together with at least another compound selected from the group consisting of vitamin E, pentoxifylline and interferon-gamma, in particular gamme-interferon-1-b.

The invention also describes and relates to a method of treating a patient receiving, having received or set to receive radiotherapy against breast cancer, comprising the steps of providing a Rho kinase inhibitor to said patient, and optionally (but preferably) another drug useful for treating or preventing fibrosis.

Also comprised are methods for treating fibrosis induced by radiotherapy, comprising the steps of administering to a patient in need thereof therapeutic doses of a Rho kinase inhibitor and of another drug against fibrosis. Said administration may be simultaneous, separate or sequential. Also comprised are methods for preventing onset of fibrosis induced by radiotherapy, comprising the steps of administering to a patient in need thereof therapeutic doses of a Rho kinase inhibitor and of another drug against fibrosis. Said administration may be simultaneous, separate or sequential.

In one particular embodiment which is preferred (in particular for problems of ease of use by the patient), the Rho kinase inhibitor is in a form suitable for oral administration. This therefore involves a composition for oral administration, which will contain at least or about or exactly 5 mg, preferably at least or about or exactly 10 mg of the Rho kinase inhibitor, or even at least or about or exactly 20 mg, at least or about or exactly 40 mg, at least or about or exactly 50 mg of the Rho kinase inhibitor. This composition is preferentially for pharmaceutical use, and is therefore a medicine. It is understood that each unit dose of this composition contains at least or about or exactly 5 mg, preferably at least or about or exactly 10 mg, or even at least or about or exactly 20 mg, at least or about or exactly 40 mg, at least or about or exactly 50 mg of the Rho kinase inhibitor, as active ingredient.

The total dose of the Rho kinase inhibitor may be administered once a day, or through multiple intakes. In particular, the Rho kinase inhibitor may be taken through two or three intakes a day. If provided at multiple times during the day, it is preferred when the amount of the Rho kinase inhibitor is substantially the same for each intake. It is however preferred to have a single intake of the Rho kinase inhibitor, in particular when it is a statin.

In one particular embodiment, this composition for oral administration contains the Rho kinase inhibitor as sole active ingredient, and also excipients, without any other active ingredient. An excipient should be understood to mean any compound being part of the formulation which is intended to act as a simple support, i.e. which is not intended to have a biological activity.

Excipients which can be used by those skilled in the art are well known in the art. Talc (E553b), microcrystalline cellulose (E460), lactose, Polyvinylpyrrolidone (E1201), mannose, starch (in particular corn starch), crosslinked carboxymethyl cellulose (E468), magnesium oxide (E530), iron oxide (E172), magnesium stearate (E572) and stearic acid (E570) can thus be chosen. This list is not exhaustive.

This composition can be in any form known in the art. In particular, it is in the form of a solid composition such as gel capsules, tablets (optionally film-coated), pills or lozenges. In another embodiment, it is in the form of a liquid composition such as a syrup.

A slow release (or slow sustained) form may also be envisaged. Said slow release compositions are known in the art and described in particular in WO 2011/077239. In particular, said slow release compositions may comprise a slow release matrix comprising the Rho kinase inhibitor alone or with one or more active ingredient(s).

In a specific embodiment, the slow release composition comprises a matrix allowing immediate release, wherein said matrix comprises the Rho kinase inhibitor alone or with one or more other active ingredient(s) and the slow release is achieved by a release modifying matrix or coating.

Thus, the slow release composition may provide immediate release and differed (slow) release of the Rho kinase inhibitor.

In a specific embodiment, slow release may be achieved through an osmotically driven release system.

In another embodiment, the slow release composition comprises a core comprising the Rho kinase inhibitor, optionally one or more active ingredient(s), and optionally pharmaceutical excipient(s) and one or more outer layers, wherein the outer layers comprise one or more slow release agent(s).

In another aspect, although not preferred, the Rho kinase inhibitor may be in the form which allows administration by injection. The excipients that can be used for the production of injectable compositions are well known in the art. Mention may in particular be made of sodium dihydrogen phosphate, sodium bicarbonate (E550i), methyl para-hydroxybenzoate (E218) and propyl para-hydroxybenzoate (E216), which can be used together in proportions that those skilled in the art are capable of determining. The water used is water for injection. The injection is preferably carried out intramuscularly. It can also be carried out intravenously.

It is to be noted that the fibrosis herein described is the consequence of the RT of the breast for treating the cancer. It is preferable that treatment with the Rho kinase inhibitor to start before the radiotherapy and to the continued for some time after the end of the radiotherapy, in order to be the most effective, to prevent the occurrence of fibrosis at the time the patient's tissue are damaged or are recovering.

The invention also relates to a method for treating a patient set to receive a radiation therapy for breast cancer comprising the steps of
(a) determining the rate of lymphocytes having undergone apoptosis upon irradiation for said patient
(b) determining whether said patient has smocking habit or not
(c) determining whether said patient receives a hormonotherapy
(d) calculating the individual risk of said patient to develop a fibrosis with the use of the Cox function as disclosed above,
(e) starting a treatment of said patient with a Rho kinase inhibitor prior to starting the radiation therapy for said patient if the risk is higher than a predetermined value.

In a preferred embodiment, treatment with the Rho kinase inhibitor is started if the risk of developing BLE is higher than 8 %.

The invention also relates to a method for determining whether a patient should receive a preventive treatment against fibrosis, comprising the steps of
(a) determining the rate of lymphocytes having undergone apoptosis upon irradiation for said patient
(b) determining whether said patient has smocking habit or not
(c) determining whether said patient receives a hormonotherapy
(d) calculating the individual risk of said patient to develop a fibrosis with the use of the Cox function as disclosed above,
wherein said patient should receive a preventive treatment against fibrosis if the risk calculated in (d) is higher than a predetermined value.
In a preferred embodiment, said predetermined value is higher than 8 %.

### Examples

### Example 1 - Procedure for Radiation-induced CD8 T-lymphocyte Apoptosis (RILA)

RILA assay commonly comprises the following steps:
a) cell culture of blood samples
b) sample irradiation at 8Gy using a linear accelerator, and
c) sample labelling for evaluation of T-lymphocytes apoptosis, in particular with FACS analysis.

The man skilled in the art may use classical methods to manage the RILA assay.

The evaluation of T-lymphocytes apoptosis at the step c) may use alternative methods such as dosage of Anexin 5, dosage of caspases or FACS analysis, preferably FACS analysis.

Preferred protocols are as follows:
Cell culture of blood samples:
   - Before radiotherapy (RT) one blood sample is collected from each patient in heparinized tubes and a volume of blood is aliquoted into well plate;
   - The Assay is generally carried out in triplicate (0 Gy and 8 Gy).
   - A culture medium is added (ex: add 2 ml of medium RPMI1640 (Gibco), supplemented with 20% FCS) per well in 6 well tissue culture plates.
   - Place the tissue culture plates in culture for 24 hours at 37°C, 5% CO2.

Sample irradiation at 8Gy using a linear accelerator:
- After 15 to 30 hours of culture, in particular after 24h, the plates are safely transported to the irradiator and handle to a radiation technologist, for applying an irradiation of 8Gy with a dose rate of 1Gy/min.

In the preferred embodiment, the conditions of the irradiation are:
Dose rate = 1Gy/min
Energy = 6MV
Depth = 15 mm polystyrene on cells.
DSP (Distance between Source and Plate) = 145cm
Field = 25 * 25cm ² collimator.
Linear accelerator (machine output: 200UM/mn): 200UM for 1Gy: 1600UM for 8Gy.
   - Control cells are removed from the incubator and placed for the same period of time but without radiation treatment.
   - plates are incubated for 40 to 50 hours at 37°C, 5% CO2.

Sample labelling for evaluation of T-lymphocytes apoptosis, in particular with FACS analysis:
- after 40 to 50 hours incubation, in particular 48h of incubation, the plates are removed from the incubator and placed at room temperature;
- the content of each well is transferred to pre-labeled centrifuges tubes, and after centrifugation (ex: 5mn at 1450rpm), the content is labeled with anti-human CD4 and/or CD8-FITC antibody, preferably CD8-FITC antibody;
- after addition of lysis buffer, reagents are added to each tube for evaluating lymphocytes apoptosis according to usual methods known from the man skilled in the art, in particular according to flow cytometry method (FACS) with propidium iodide and RNase A as reagents.

### Example 2 - addition of tobacco smoking habits and adjuvant hormonotherapy

Multivariate models shows that three parameters (RILA, tobacco smoking and adjuvant hormonotherapy) are independent parameters with an increased risk of BLE for active/former tobacco smoking patients (HR=1.57 CI95%[0.939-2.625]) and for patients treated by hormonotherapy (HR=3.10 CI95%[1.327-7.243]) and a decrease of risk for elevated level of RILA (HR=0.96 CI95% [0-926-0-990]).

The other clinical parameters (age, boost and node irradiation) may also be integrated in multivariate model for adjustment because of clinically relevance, although these parameters are generally not selected for definitive model, for ease of calculation of the risk, as it reduces the number of information needed for calculating the risk.

The combination of the parameters (tobacco smoking habits, adjuvant hormonotherapy and RILA) allowed prediction of the probability to develop a breast late effect, with an excellent negative predictive value (more than 90%).

## Claims

1. Rho kinase inhibitor for use thereof in the treatment of fibrosis induced by breast cancer radiotherapy.

2. Rho kinase inhibitor for use thereof for prevention of fibrosis induced by breast cancer radiotherapy.

3. Rho kinase inhibitor for use according to claim 1 or 2, wherein the patients have a percentage of lymphocytes undergoing apoptosis upon irradiation lower than 20 %.

4. Rho kinase inhibitor for use according to claim 1 or 2, wherein the patient has a probability to develop Breast Late Effect higher than 8 % as established by an *in vitro* method comprising the steps of:
a. Determining the percentage of lymphocytes undergoing apoptosis upon irradiation the patient, from a biologic sample of said subject, preferably a blood sample of said subject;
b. Determining the tobacco smoking habit and presence of adjuvant hormonotherapy in the patient;
c. Combining said data through a multivariate Cox function to obtain an end value to determine the risk of developing BLE;
wherein the multivariate Cox function is obtained from the results obtained from a cohort of at least 100 patients, through the following steps
i) Assessing the presence of BLE in each patients, wherein the following data are known for each patient
a. The RILA result
b. The tobacco smoking habits
c. The presence of adjuvant hormonotherapy; and
ii) Performing a multivariate Cox regression by combination of said data a., b. and c.,
iii) Analyzing said multivariate Cox regression to assess the independent discriminative value of said data a., b. and c.,
iv) Combining the relative weight of each data a., b. and c., as individually determined in the multivariate Cox regression, with a negative sign when the markers harbor a negative correlation with the observation of BLE.

5. Rho kinase inhibitor for use according to any one of claims 2 to 4, in which the Rho kinase inhibitor is administered to the patients during the radiotherapy treatment.

6. Rho kinase inhibitor for use according to any one of claims 2 to 5, in which the treatment with the Rho kinase inhibitor has already been started at the time of initiation of the radiotherapy treatment.

7. Rho kinase inhibitor for use according to any one of claims 2 to 6, in which the treatment with the Rho kinase inhibitor is continued after termination of the radiotherapy treatment.

8. Rho kinase inhibitor for use according to any one of claims 1 to 7, wherein the Rho kinase inhibitor is a HMG-CoA reductase inhibitor.

9. Rho kinase inhibitor for use according to claim 1 to 8, wherein the Rho kinase inhibitor is pravastatin.

10. Rho kinase inhibitor for use according to claim 9, in which the daily amount of pravastatin administered to the patient is comprised between 10 and 50 mg.

11. Rho kinase inhibitor for use according to any one of claims 1 to 10, which is in a form suitable for oral administration.

12. Rho kinase inhibitor for use according to any one of claims 1 to 11, which is in the form of gel capsules, tablets (optionally film-coated), lozenges or pills.

13. Rho kinase inhibitor for use according to any one of claims 1 to 12, which is in the form of a slow release composition.

14. Composition containing a Rho kinase inhibitor and another drug against fibrosis for simultaneous, separate or sequential (spread out over time) use in the treatment or prevention of fibrosis induced by radiotherapy.

15. Composition according to claim 15, wherein said other drug selected from the group consisting of vitamin E, pentoxifylline and interferon-gamma-1 b.
